# EUROPEAN PATENT APPLICATION

(11) **EP 4 023 144 A1**
(43) Date of publication of application: **06.07.2022**
(21) Application number: 20217768.9
(22) Date of filing: 30.12.2020
(51) Int. Cl.: A61B 5/00, A61B 5/1455

(54) **NIRS DEVICE AND METHOD**

(71) Applicant: NIRx Medizintechnik GmbH, 13355 Berlin (DE)
(72) Inventor: von LÜHMANN, Alexander, 13355 Berlin (DE); BRITZ, Patrick, Gustav-Meyer-Allee 25 13355 Berlin (DE)
(74) Representative: Maikowski & Ninnemann Patentanwälte Partnerschaft mbB

(57) **Abstract**

The invention relates to a NIRS device (10) with a plurality of NIR emitters (2) for emitting light (EL) and a plurality of NIR detectors (3) for receiving light (RL), the NIR emitters (2) and NIR detectors (3) configured to be coupled with a device (1) to be worn by a person (50), the NIR detectors (3) generating NIR measurement signals (NMS) in response to the received light (RL), comprising
at least one ambient electromagnetic detector (5) configured to detect ambient electromagnetic signals (L) and to generate an ambient measurement signal (AMS) dependent of the ambient electromagnetic signals (L) measured,
a data processing unit (20) processing the ambient measurement signal (AMS), and
an adapting means (21) of the data processing unit (20) for adapting at least one property of the emitted light (EL) in dependence of the ambient measurement signal (AMS) to reduce or minimize the effect of the ambient electromagnetic signals (L), in particular in output data (O) of the data processing unit (20). The invention also relates to a NIR spectroscopic method.

## Description

The invention relates to a NIRS device with the features of claim 1 and a NIRS method with the features of claim 15.

NIRS (near infra-red spectroscopic) devices have been used in the scientific and medical investigation of tissue and brain oxygenation for quite some time, as e.g. described for functional NIR spectroscopy of the brain in the article Scholkmann et al., A review on continuous wave functional near-infrared spectroscopy and imaging instrumentation and methodology", Neurolmage, 85 (2014), 6-27.

In the following NIR devices, such as functional near-infrared spectroscopy (fNIRS) devices or functional near-infrared imaging (fNIRI) devices, will be termed as "NIRS device".

For this application, a plurality of NIR emitters is coupled with a device to be worn by a person. The NIR emitters emit light (e.g. by a laser or LED having a controlled frequency and / or intensity) into a part of the body, such as e.g. the head, in particular the brain of the person. This enables e.g. the measurement of the oxygenation in the brain tissue.

In some cases, two or more discrete wavelengths are used in the emitted light. Typical wavelengths for this light are 695 nm, 730 nm or 850 nm as they are in an optical wavelength window (approximately from 550 to 980 nm) in which the NIR absorption in water, protein, fat or collagen is relatively different from the NIR absorption by oxygenated Hemoglobin (O₂Hb) or by deoxygenated Hemoglobin (HHb). In other cases, a continuous portion of the NIR spectrum is emitted into the head of the person.

A plurality of NIR detectors is placed on the same device to be worn on the body to receive light signals that have interrogated the underlying tissue between NIR emitters and detectors. The light signals undergo scattering and absorption events in the tissue that depend on physiological changes, for instance changes in oxygenation and blood flow in brain tissue. Typically, silicon-based photodiodes, avalanche photodiodes, Single-photon avalanche diodes (SPADs), Silicon Photomulitipliers (SiPM) or photomultiplier tubes are used as NIR detectors. The signals received by the NIR detectors are considered as NIR measurement signals and are then further processed to derive information about the condition (here the oxygenation) of the brain tissue. Some methods used in that respect are e.g. described in the article by Scholkmann et al..

One known disturbance to the NIR measurements signals is ambient light. Daylight, incandescent light, light from LED lamp devices, halogen light or fluorescent light are examples of ambient light. This also includes active external sensors that use NIR light to collect environmental information, such as motion tracking cameras, or Virtual Reality Light-Houses or Light Detection and Ranging (LIDAR) applications. The detrimental influence of the ambient light is, e.g., caused by the introduction of additional light intensities and / or a disturbance of the spectral data in the NIR measurement signals.

Shielding the NIR detectors from ambient light might be possible but difficult to implement in a device, such e.g. as headmount, a cap to be worn by a person or another device applied to tissue surface of the person.

The article by Sherkat et al. "SHADE: Absorption spectroscopy enhancement with ambient light estimation and narrow band detection", Optik, 220 (2020), 165116, addresses the issue of ambient light by using a software-estimator for the ambient light in the data processing.

NIR measurements are increasingly performed in a natural setting, i.e. outside a laboratory setting, to evaluate the brain functionality of a person under natural or real working conditions. Therefore, the interference of ambient light - and other ambient electromagnetic signals - with the NIR measurements needs to be addressed efficiently.

This is addressed by a NIRS device with the features of claim 1.

The NIRS device comprises a plurality of NIR emitters for emitting light and a plurality of NIR detectors for receiving light reflected from the brain tissue. For measuring neurological data from a person's brain, the NIR emitters and NIR detectors are configured to be coupled with a cap to be worn by a person. The NIR detectors are generating NIR measurement signals in response to the received light. The emitted light is e.g. in the range of 550 to 980 nm.

The NIRS device further comprises at least one ambient electromagnetic detector (e.g.an ambient light detector) which is configured to detect ambient electromagnetic signals and to generate an ambient measurement signal AMS dependent on the measured ambient electromagnetic signals measured. This can e.g. be a separate measurement channel from the NIR detectors. It should be noted that the ambient electromagnetic signals comprises electromagnetic interference signals. The ambient electromagnetic detector detects analog signals, such as e.g. ambient light in the wavelength range of 550 to 980 nm.

In one embodiment the ambient measurement signal and the NIR measurement signals can be measured in separate channels. But it is also possible have the both signals in one channel as they can be separated e.g. through a spectral analysis.

A data processing unit processes the ambient measurement signal AMS and an adapting means of the data processing unit is configured for adapting at least one property of the emitted light in dependence of the ambient measurement signal AMS. This results in a reduction or minimization of the effect of the ambient electromagnetic signals, in particular on the output data the data processing unit. The output data is generated by the data processing unit using the NIR measurement signal NMS. The output data can e.g. be used in a neuroimaging device to visualize the oxygenation of certain regions in the brain or in oximetry device in general for other body parts.

In one embodiment, at least one property of or derived from the ambient electromagnetic signal (e.g. ambient light signal) is used as a reference signal in a lock-in device. For example, a frequency spectrum measured by the ambient electromagnetic signal detector can be used to identify a reference signal frequency fₘᵢₙ that minimizes the overlap of spectral energies with ambient noise used in the NIR measurement signal NMS. With this, a clear indication of the ambient electromagnetic signal, such as e.g. an ambient light signal is identified so that the adaptation of the emitted light can be efficiently performed.

The emitted light can, in one embodiment, comprise a continuous wave, in particular a sine wave, as typically applied in Continuous Wave NIRS, Frequency Domain NIRS or a time domain NIRS signal, in particular an impulse. All these forms of emitted light can generate information about the oxygenation of tissue, e.g. of brain tissue.

In particular, a modulation and / or demodulation frequency in the emitted light is adaptable by the adapting means to reach a level in which there is less interference with the ambient electromagnetic signal, e.g. the ambient light.

In a further embodiment, the adapting means is configured to choose a frequency band, in particular the frequency band for the emitted light with the least interference with the ambient electromagnetic signals, e.g. by identifying a local or global minimum within the investigated frequency band of the ambient noise power spectrum.

The adaptation means can be also configured to choose a frequency for the emitted light or a frequency band for the emitted light with an interference below a predetermined threshold.

The adapting means can further be configured to adapt the frequency or the frequency band of the NIR emitters in the digital domain or in the analog domain.

In a further embodiment, the intensity of the emitted light as continuous wave or the emitted light in the time domain is adapted by the adapting means. This means, e.g., the reference signal can be generated by an intensity signal which can be distinguished from the ambient electromagnetic signal, e.g. ambient light.

At least one of the ambient electromagnetic detectors can be spatially separated from the NIR detectors.

It is also possible that at least one of ambient electromagnetic detectors is integrated with a NIR detector.

In one embodiment, the device to be worn on the body or a body part is configured as a cap, a headmount, a helmet, a clip device to attach to a finger or an ear lobe or a patch to be worn on the skin. With this it is possible, to use the NIRS device in a wide range of measurement situations.

One application of an embodiment is that the NIRS measurements signals (NMS) are used in an oximetry measurement in the tissue of a person.

The issue is also addressed by a method with the features of claim 15.

Embodiments are shown in the following in the context of the figures.
- Fig. 1: shows a first embodiment of a spectroscopic device;
- Fig. 2: shows a second embodiment of a spectroscopic device;
- Fig. 3: shows a first embodiment of the adaptation means using a minimum search in the Power Spectral Density of the Ambient Noise Signal AMS.

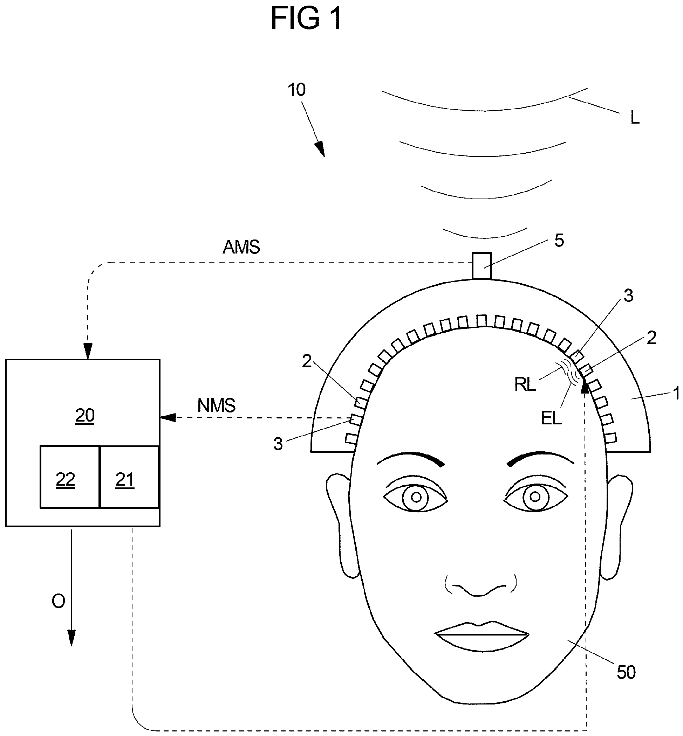

In Fig. 1, a NIRS device 10 is schematically shown. The purpose of the NIRS device 1 is the measurement of the oxygenation of the brain tissue in the head of a person 50. The oximetry of brain tissue is just an example application of the NIRS device 10.

In this embodiment the person 50 wears a headmount or a cap 1 on the head comprising a plurality of NIR emitters 2 and NIR detectors 3 arranged in a pattern over the surface of the cap 1. For reasons of simplicity the wiring of the NIR emitters 2 is not shown here.

The cap 1 is just an example of a device 1 to be worn on the body of the person 50, the head of the person being such a body part. In principle the NIR detectors 3 can be used in connection with other body parts in which NIR signals are to be measured. This can be e.g. a clip device to be worn on a finger or an ear lobe. Also a patch worn on the skin of the person 50 would be an embodiment of the device 1.

The NIR emitters 2 in this embodiment are configured as LED emitters with discrete wavelengths of 760 and 850 nm. In alternative embodiments, NIR Laser emitters or other continuous NIR emitters could be used. Also, different wavelengths within the known wavelength window can be used. In principle, the NIR emitters 2 can emit light EL as continuous intensity signals, as modulated signals in the frequency domain or as discrete pulsed signals in the time domain.

The NIR emitters 2 emit the light EL through the scalp of the head and through the bone of the skull into the brain tissue. Typically, the emitted light EL can penetrate about 15 mm into the brain tissue.

Reflected light RL is detected by a plurality of NIR detectors 3 also coupled with the cap 1 in a certain pattern and with known distances to the NIR emitters 2. Again, the wiring of the NIR detectors 3 is not shown here for the sake of simplicity.

Upon the detecting the reflected light RL from the brain tissue, the NIR detectors 3 generate NIR measurement signals (NMS) which can then be processed by a data processing unit 20 in a generally known way.

The data processing unit 20 generates output data O comprising data related to the oxygenation of the brain tissue which then can e.g. be visualized in a generally known way.

In the embodiment shown here, the NIR detectors 3 are silicon photodiodes. In other embodiments, silicon photomultiplier (SiPM), single-photon avalanche diode (SPAD), avalanche photodiodes in general or photomultiplier tubes can be used as NIR detectors 3.

The number of NIR emitters 2 and NIR detectors 3 coupled to the cap 1 can be in the range from 1 to more than 50, respectively.

The issue is that ambient light L - as one possible form of ambient electromagnetic signals - can disturb the NIR measurement signals NMS. In the embodiment of Fig. 1 an ambient electromagnetic detector 5 (e.g. an ambient light detector) is used to measure the ambient electromagnetic signals L such as daylight or artificial light to which the person 50 is subjected to.

As mentioned above, the ambient electromagnetic signal L will interfere with the measurements taken by the NIR detectors 3. Due to the many NIR detectors 3 positioned on a complex surface, such as the head, some ambient electromagnetic signal L will be detected by the NIR detectors 3 increasing the noise in the NIR measurement signals.

The data processing unit 20 is also receiving the ambient measurement signal AMS from the ambient electromagnetic detector 5. This data is generally independent from the NIR measurement signals in this embodiment.

The data processing unit 20 comprises adapting means 21 for adapting the emitted light EL dependent on the ambient measurement signal AMS to reduce or minimize the effect of the ambient electromagnetic signals L, in particular in the output data O of the data processing unit 20. This adaptation of the emitted light is indicated in Fig. 1 as a dashed line.

Therefore, the modulation frequency of the emitted light EL can in one embodiment be modified by the adapting means 21 with a fixed but adjustable frequency in the digital domain or the frequency domain.

The adapting means 21 can use data derived from at least one property of the ambient measurement signals AMS as a reference signal for a lock-in detector 22 coupled to the data processing unit 20.

In general, lock-in detectors 22 are used to measure the frequency and phase of a signal (here the NIR measurement signal NMS) relative to a defined reference signal (here a signal derived from the ambient measurement signals AMS). The lock-in detector 22 can extract a signal with a known reference signal even if the measured signals (here NMS) is extremely noisy.

In the present context, the ambient measurement signals AMS are measured by the ambient electromagnetic detector 5 separately from the reflected light RL which is detected by NIR detectors 3.

The effect of the ambient electromagnetic signal L can be seen in the NIR measurement signal NMS, but as the ambient measurement signals AMS can be used as a reference signal, the lock-in detector 22 can find this effect and react on it, here by an adaptation of the emitted EL generated by the NIR emitter 2.

The adapting means 21 of the data processing unit 20 can adapt at least one property of the emitted light EL from the NIR emitters 2 in dependence of the ambient measurement signal AMS to reduce or minimize the effect of the ambient electromagnetic signals L in the output data O of the data processing unit 20. The adapting means 21 can e.g. use the reference signal derived from at the least one property of the ambient measurement signal AMS that is also used as a reference signal for a lock-in detector 22 as a reference signal for the NIR emitter 2.

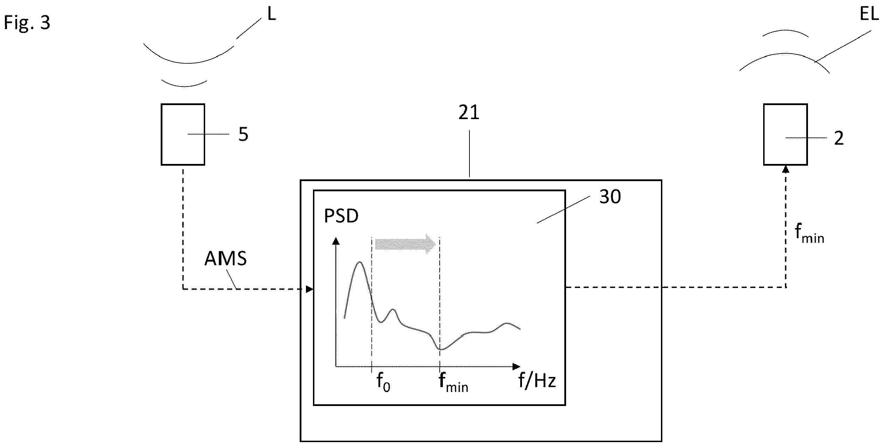

In one embodiment, at least one property of the emitted light EL is adapted in a way so that the interference with the ambient light L is reduced or minimized. For example, the frequency or wavelength of the emitted light and / or the amplitude of the emitted light EL can be shifted to values which allow for a better signal-to-noise ratio in respect to the ambient light L (see e.g. Fig. 3).

As the embodiment is measuring the ambient electromagnetic signals L over time (either discretely or continuously) changes in the dynamic ambient electromagnetic signals L will be dynamically adapted in changes in the emitted light EL.

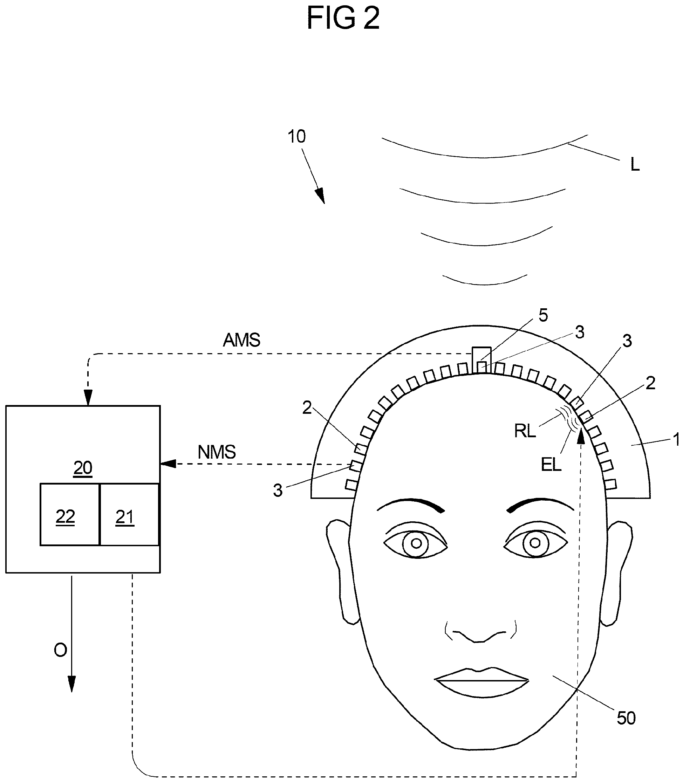

In Fig. 2, a variation of the embodiment shown in Fig. 1 is described so that reference can be made to the respective description.

In the embodiment of Fig. 1, the ambient electromagnetic detector 5 was a distinct unit, separate from the NIR detectors 3. In the embodiment shown in Fig. 2, the ambient electromagnetic detector 5 is integrated in the unit of the NIR detector 3, but it still provides a separate measurement of the ambient light L from the measurement of the reflected light RL.

In the embodiments shown in Fig. 1 and 2 the ambient measurement signal AMS and the NIR measurement signals NMS are processed separately which allows an efficient data handling. In principle it is possible that the ambient measurement signal AMS and the NIR measurement signals NMS are jointly measured (with a sufficiently high sampling rate) and processed as one signal. A signal processing method (e.g. using an FFT) can be used to separate the two signals, by evaluating the modulation peak in the power density spectrum. It is in principle possible to detect a sub-optimal band (i.e. the peak around the modulation frequency in the power density spectrum is rather broad) and consequently shift the modulation frequency towards a part with a lower energy in the power density spectrum.

In Fig. 3, an embodiment of the adapting means 21 is described, in which the spectral content (Power Spectral Density, PSD) of the AMS signal is being analyzed within the frequency band of interest to identify a region of low interference.

If the original lock-in modulation/demodulation reference signal f₀ is identified to be in a non-optimal region (e.g. AMS band with high noise power), it is being adapted by shifting it to a better, or even optimal region (i.e. AMS band with low noise power) at fₘᵢₙ.

### Reference numbers

- 1: device to be worn on the body of a person with NIR emitters and NIR detectors, e.g. a cap
- 2: NIR emitter
- 3: NIR detector
- 5: ambient electromagnetic detector, e.g. ambient light detector
- 10: NIRS device
- 20: data processing unit
- 21: adapting means for NMS measurement signals
- 22: lock-in detector
- 30: Means to analyze the power spectrum of an AMS or RL signal. PSD: Power Spectral Density
- 50: person
- AMS: Ambient measurement signal
- EL: emitted light
- L: ambient electromagnetic signals (e.g. ambient light)
- NMS: NIR measurement signal
- O: output data
- RL: reflected light

## Claims

1. NIRS device (10) with a plurality of NIR emitters (2) for emitting light (EL) and a plurality of NIR detectors (3) for receiving light (RL), the NIR emitters (2) and NIR detectors (3) configured to be coupled with a device (1) to be worn by a person (50), the NIR detectors (3) generating NIR measurement signals (NMS) in response to the received light (RL), comprising
at least one ambient electromagnetic detector (5) configured to detect ambient electromagnetic signals (L) and to generate an ambient measurement signal (AMS) dependent of the ambient electromagnetic signals (L) measured,
a data processing unit (20) processing the ambient measurement signal (AMS), and
an adapting means (21) of the data processing unit (20) for adapting at least one property of the emitted light (EL) in dependence of the ambient measurement signal (AMS) to reduce or minimize the effect of the ambient electromagnetic signals (L), in particular in output data (O) of the data processing unit (20).

2. NIRS device (10) according to claim 1, wherein the ambient electromagnetic detector (5) comprises a sensor for ambient light, in particular in the range of 550 to 980 nm.

3. NIRS device (10) according to claim 1 or 2, wherein the ambient measurement signal (AMS) and the NIR measurement signals (NMS) are measured in separate channels.

4. NIRS device (10) according to at least one of the preceding claims, wherein at least one property of or derived from the ambient electromagnetic signals (L) is used as a reference signal in a lock-in device (22).

5. NIRS device (10) according to at least one of the preceding claims, wherein the emitted light (EL) comprises a continuous wave NIR signal, a frequency domain wave NIR signal, in particular a sine wave or a time domain NIR signal, in particular an impulse.

6. NIRS device (10) according to at least one of the preceding claims, wherein a modulation and / or demodulation frequency in the emitted light (EL) is adaptable by the adapting means (21).

7. NIRS device (10) according to at least one of the preceding claims, wherein the adapting means (21) is configured to choose a frequency band in the emitted light (EL), in particular a frequency band for the emitted light (EL) with the least interference with the ambient electromagnetic signals (L).

8. NIRS device (10) according to at least one of the claims 1 to 6, wherein the adapting means (21) is configured to choose a frequency of the emitted light (EL) or a frequency band for the emitted light (EL) with an interference below a predetermined threshold.

9. NIRS device (10) according to at least one of the preceding claims, wherein the adapting means (21) is configured to adapt the frequency or the frequency band of the NIR emitters (3) in the digital domain or in the analog domain.

10. NIRS device (10) according to at least one of the preceding claims, wherein the intensity of the continuous wave or the time domain signal (EL) emitted by the NIR emitter (2) is adapted by the adapting means (21).

11. NIRS device (10) according to at least one of the preceding claims, wherein at least one of the ambient electromagnetic detectors (5) is spatially separate from the NIR detectors (3).

12. NIRS device (10) according to at least one of the preceding claims, wherein at least one of the ambient electromagnetic detectors (5) is integrated with an NIR detector (3).

13. NIRS device (10) according to at least one of the preceding claims, wherein the device (1) to be worn on the body or a body part is configured as a cap, a headmount, a helmet, a clip device to attach to a finger or an ear lobe or a patch to be worn on the skin.

14. NIRS device (10) according to at least one of the preceding claims, wherein the NIRS measurements signals (NMS) are used in an oximetry measurement in the tissue of a person.

15. NIRS method using a plurality of NIR emitters (2) for emitting light (EL) and a plurality of NIR detectors (3) for receiving light (RL), the NIR emitters (2) and NIR detectors (3) configured to be coupled with a device (1) to be worn by a person (50), the NIR detectors (3) generating NIR measurement signals (NMS) in response to the received light (RL), wherein
a) at least one ambient electromagnetic detector (5) detects ambient electromagnetic signals (L) and generates an ambient measurement signal (AMS) dependent on the ambient electromagnetic signals (L) measured,
b) a data processing unit (20) processes the ambient measurement signal (AMS), and
c) an adapting means (21) of the data processing unit (20) adapts at least one property of the emitted light (EL) in dependence of the ambient measurement signal (AMS) to reduce or minimize the effect of the ambient electromagnetic signals (L), in particular in output data (O) of the data processing unit (20).
